# EUROPEAN PATENT SPECIFICATION

(11) **EP 2 548 507 B1**
(45) Date of publication and mention of the grant of the patent: **26.11.2014**
(21) Application number: 10847831.4
(22) Date of filing: 28.12.2010
(51) Int. Cl.: A61B 5/154, A61B 5/15, A61B 5/153

(54) **VACUUM BLOOD COLLECTION TUBE, BLOOD COLLECTION UNIT AND DEVICE FOR DISCRIMINATING TEST METHODS**
VAKUUMBLUTSAMMELRÖHRCHEN, BLUTSAMMELEINHEIT UND VORRICHTUNG ZUR UNTERSCHEIDUNG VON TESTVERFAHREN
TUBE DE PRÉLÈVEMENT SANGUIN SOUS VIDE, UNITÉ DE PRÉLÈVEMENT SANGUIN ET DISPOSITIF POUR DISCRIMINER DES MÉTHODES D'ESSAI

(30) Priority: 15.03.2010 JP 2010057568
(43) Date of publication of application: 23.01.2013
(73) Proprietor: Matumura, Takahito, Tokyo 162-0844 (JP)
(72) Inventor: Matumura, Takahito, Tokyo 162-0844 (JP)
(74) Representative: Epping, Wilhelm
(86) International application number: PCT/JP2010/007618
(87) International publication number: WO 2011/114413

(56) References cited:
- JP-A- 1 032 168
- JP-A- 2 004 337
- JP-A- 6 007 328
- JP-A- 7 051 251
- JP-A- 11 076 205
- JP-A- 63 216 542
- JP-A- 63 296 733
- JP-A- 2002 502 277
- JP-A- 2008 284 400
- US-A- 4 150 089

## Description

### Technical Field

The present invention relates to a vacuum blood collection tube, a blood collection unit using the vacuum blood collection tube and a device for discriminating test methods for a blood collected by the vacuum blood collection tube.

### Background Art

Blood collection methods used in the medical fields or the like include two methods, one using a syringe and another using a vacuum blood collection tube. The blood collection methods using the syringe require dispensation of the blood collected for conducting tests, has low working efficiency and is at a risk for infection. Consequently, blood collection methods using the vacuum blood collection tube is currently being mainly used.

The vacuum blood collection tube includes a bottomed tubular body and a plug body which hermetically seals an open end of the tubular body in order to maintain an inner space of the tubular body at a prescribed reduced pressure. The blood collection unit using this vacuum blood collection tube has not only the vacuum blood collection tube, but also at least the blood collection needle and a bottomed cylindrical holder which holds the vacuum blood collection tube. The blood collection needle is normally formed of a hollow metal narrow tube one end of which is punctured into a blood vessel and the other end of which is punctured into the plug body of the vacuum blood collection tube, and has a needle hub with a cut male screw on an approximately central part in a longitudinal direction. The blood collection needle is screwed into a needle-joining part in which the needle hub is provided with the male screw cut, on the bottom of the holder, and the other end is fixed so as to project in the holder. In blood collection procedure, first, the one end of the blood collection needle is punctured into the blood vessel in this state, the vacuum blood collection tube is inserted from the plug body side to the open end of the holder, the plug body is punctured by another one of the blood collection needle, and the vacuum blood collection tube is pushed thereinto until the other end is exposed on the inside of the tubular body. Subsequently the blood is collected in the inner space by a differential pressure between a blood pressure and a pressure of the inner space and flows thereinto until the blood pressure and the pressure of the inner space reach a state of equilibrium. In a case of sequential blood collection, when the state of equilibrium is reached and inflow of the blood is discontinued, the vacuum blood collection tube is pulled from the holder, and a new vacuum blood collection tube is pushed thereto in the similar manner.

In a case of a blood test, the test includes a plurality of different test items, biochemistry, blood glucose level, blood count, etc., per one blood collection subject. Each test usually requires different processes such as an amount of blood required for the test, necessity of an anticoagulant agent and necessity of upside-down flipping. Therefore, according to the number of the test items, the sequential blood collection is to be conducted as stated above. When the tube is exchanged for a new vacuum blood collection tube for the sequential blood collection, the vacuum blood collection tube must be pushed into the holder again, therefore the blood collection needle punctured into the blood vessel also moves in a direction of puncture by receiving influence of this pushing force. Consequently, the blood collection subj ect feels a pain on each exchange for a new vacuum blood collection tube. That is, the blood collection subject feels not only a pain in first puncture by the blood collection needle but also pains of the number of the test items. Additionally, since a punctured state is continued for a long time compared to usual preventive injection or the like, the blood collection subject physically and mentally feels stressed. In addition, when the blood is simultaneously collected for a large number of the blood collection subjects in mass examination, a disaster site, a battlefield, etc., a plurality of blood test items lay a heavy burden on persons who sample the blood such as nurses, resulting in problems of lowered working efficiency.

Conventionally, a blood collection method has been particularly proposed, wherein the vacuum blood collection tube constituted so that a blood collection tube body which houses the collected blood for reducing physical burden on the blood collection subject and an outer cylinder which houses the blood collection tube body movably in an axis direction are able to move relative to the axis direction is used, and the blood count is measured while the blood collection tube body is housed in the outer cylinder, and then an erythrocyte sedimentation rate is measured while an approximately half part from the end of the blood collection tube body projects from the outer cylinder. According to this constitution, measurements of the blood count and the erythrocyte sedimentation rate could be conducted in one blood collection procedure, and the physical burden on the blood collection subject could be reduced.

### Citation List

### Patent Literature

PTL 1: Japanese Patent Laid-Open No. 2005-156332
PTL 2: United States Patent US 4,150,089 A

### Summary of Invention

### Technical Problem

In relation to the above prior art PTL 1, because a new vacuum blood collection tube must be inserted into the blood collection tube holder in a case of three or more test items, the conventional problems in collecting the blood have not yet been solved fundamentally in that the blood collection subject feels a plurality of pains.

In document PTL 2 a multi-chamber blood collection test tube is described, which is composed of interconnected separable and separate tube segments, which are joined together for purposes of taking the sample and which are separated for conducting tests.

In addition, it is described in the above prior art that the erythrocyte sedimentation rate is measured by a blood anticoagulated with the use of EDTA-2k in measurements of the blood count. However, there have been problems in which some test items may cause troubles in the same anticoagulation, and accurate test results cannot be obtained by the above prior art because anticoagulation is not originally conducted in biochemical tests.

Incidentally, in a case where a hospital and a clinic conducting the blood collection do not have blood test apparatuses, they ask an external inspection institute for testing of the collected blood, and thus the vacuum blood collection tube is required to be transferred thereto. The vacuum blood collection tubes after the blood collection are usually made to stand in rows with their plug bodies up on dedicated stands and transferred to the inspection institute as they are. Thus, when the stands holding the vacuum blood collection tubes in rows are stacked and transferred, they are unstable, therefore there have been some problems of poor efficiency of transport in a case of transport of many tubes in the mass examination or the like.

Consequently, the object of the present invention is to provide the vacuum blood collection tube, the blood collection unit and the device for discriminating test methods, wherein each test is adequately conducted in the blood collection procedure including a plurality of test items, the pain to the blood collection subject is reduced to the minimum, and the efficiency in blood collection procedure can be improved.

### Solution to Problem

The present invention is the vacuum blood collection tube which includes the bottomed tubular body having the inner spaces with a prescribed capacity and the plug body which hermetically seals the open end of the tubular body to maintain the inner spaces at the prescribed reduced pressure, wherein the blood is collected in the inner spaces of the tubular body by the differential pressure between the blood pressure and the pressure of the inner spaces via the hollow blood collection needle one end of which is punctured into a blood vessel and the other end of which is punctured into the plug body, the inner space is divided into a plurality of inner spaces by partitions, each of the inner spaces has a prescribed capacity and maintains a prescribed reduced pressure depending on a purpose of each blood test, and the blood of an amount according to the objects of the various blood tests is collected in each of the inner spaces through the blood collection needle punctured into the vessel. Furthermore, the tubular body has, between the plug body and the plural inner spaces, a stretch membrane which allows insertion of the other end of the blood collection needle punctured into the plug body, a fixation membrane which is positioned facing the stretch membrane in a direction toward each of the inner spaces and does not allow penetration of the other end of the blood collection needle, and an accumulation area for the blood which includes an inner wall surface of the tubular body and maintains the prescribed reduced pressure, the fixation membrane is provided with communication holes which communicate the accumulation area to each of the inner spaces, and when the blood flows into the accumulation area from the other end of the blood collection needle inserted into the stretch membrane, the blood accumulates in the accumulation area while expanding the stretch membrane and divided into each of the inner spaces through the communication holes.

According to this construction, when a plurality of blood tests for different purposes is conducted, the blood can be collected in a plurality of inner spaces for each examination purpose in one blood collection procedure. Each of the inner spaces is arranged in parallel on the same plane in a shorter length direction, the tubular body is formed into a plate-like shape, and thus the tubes can be efficiently stacked and transported when they are transferred to an inspection institute or the like after the blood collection procedure.

Moreover, the blood collection unit according to the present invention is characterized mainly most by including the vacuum blood collection tube, the blood collection needle and the holder the one end of which has the opening allowing insertion into the vacuum blood collection tube and the other end of which has the needle-joining part connecting the blood collection needle. Meanwhile the blood collection needle includes a sheath body which covers the other end, and which is pierced by the other end and is contracted when the blood collection needle is stuck into the plug body of the vacuum blood collection tube inserted into the holder from the open end of the holder connected to the needle-joining part; and the other end piercing the sheath body may be constituted so as to penetrate the plug body and project to the inside of the tubular body. Furthermore, the blood collection needle has an occlusion member which occludes the other end of the blood collection needle in the tubular body and may be provided with a device configured to perform detachment, which latches the occlusion member by drawing the vacuum blood collection tube in a direction of the open end of the holder and which detaches the occlusion member from the blood collection needle. In this case, the one end of the blood collection needle is first punctured into the blood collection subject's arm, and then the blood collection needle does not move in a direction of puncture toward the vessel until the blood collection procedure is completed.

Meanwhile, in the vacuum blood collection tube according to the present invention, for example, both a blood detected by the test items in which the blood is not stirred in the test such as biochemistry and a blood detected by the test items in which the blood is stirred in performing anticoagulation treatment such as the blood glucose level are collected. Therefore, in the inner space in which the blood is not stirred, by the fact that its circumference is previously coated with an acoustic wave absorber and stirring is conducted by acoustic wave oscillation, the different treatments can be simultaneously performed.

The device for discriminating test methods according to the present invention is characterized mainly most by including a device configured to perform irradiation, which irradiates, with a prescribed light beam, a tubular body coated with given fluorescent agents respectively allowing discrimination among the respective inner spaces; a device configured to perform detection, which detects the respective fluorescence reactions of the fluorescent agents irradiated with the light beam; a device configured to perform reading-out, which reads out the test method of the blood collected in the respective inner spaces corresponding to the detected fluorescence reactions; and a device configured to perform display, which displays the test method read out. That is, since the fluorescent agents respectively show different fluorescence reactions by irradiation with the prescribed light beam, it is possible to discriminate the blood in which inner space is used for which test item, even if there is a plurality of inner spaces in the tubular body.

### Advantageous Effects of Invention

Since the vacuum blood collection tube according to the present invention allows blood collection corresponding to a plurality of test items in one blood collection procedure for the blood collection subject, there is an effect to reduce physical and mental stresses in number of pains as well as physical and mental stresses in duration of a long-term puncture for the blood collection subject.

In addition, blood collection corresponding to a plurality of test items for different purposes in one blood collection procedure is allowed, and as a result, the time required for blood collection for one blood collection subject can be shortened, therefore there is an effect to reduce a burden on a person who samples the blood such as a nurse.

Furthermore, in a case that a large number of blood samples are transferred to the inspection institute, for example in mass examination and medical examination in a temporary hospital in a disaster site or the like, etc., the tube can be efficiently stacked and transported, thereby the transport efficiency is improved, and a transportation cost can be lowered.

### Brief Description of Drawings

FIG. 1 illustrates an outline view of the blood collection unit according to the present invention.
FIG. 2A illustrates a side cross-sectional view of the blood collection unit according to the present invention, and FIG. 2B illustrates a cross-sectional view of the line A-A' in FIG. 2A.
FIG. 3A illustrates a side cross-sectional view of a state before the vacuum blood collection tube is inserted into the holder with the fixed blood collection needle, and FIG. 3B illustrates a side cross-sectional view of a state after the vacuum blood collection tube is inserted into the holder.
FIG. 4A illustrates a state where the blood collection needle is occluded by the occlusion member before the blood collection procedure, and FIG. 4B illustrates a state where the occlusion member is detached during the blood collection procedure.
FIG. 5A illustrates a top view of a non-return mechanism provided on a communication hole, and FIG. 5B illustrates its side cross-sectional view, and FIG. 5C illustrates a side cross-sectional view of the non-return mechanism while the blood inflows.
FIG. 6 illustrates a schematic view of a state where the vacuum blood collection tube according to the present invention is put into a prescribed stirrer.
FIG. 7A illustrates a drawing of a configuration in which the vacuum blood collection tube according to the present invention is formed into a plate-like shape, and FIG. 7B illustrates a drawing of its variation.
FIG. 8 illustrates a block configuration diagram of the device for discriminating test methods according to the present invention.
FIG. 9A illustrates perspective views of a cartridge-type tubular body and a tubular body capable of engaging with the cartridge-type tubular body, and FIG. 9B illustrates a bottom view in which the tubular body is engaged with the cartridge-type tubular body.
FIG. 10A illustrates a side cross-sectional view of an attachment part before the cartridge-type tubular body is attached, and FIG. 10B illustrates a side cross-sectional view of the attachment part after the cartridge-type tubular body is attached.
FIG. 11 illustrates a flow diagram which shows a procedure of the blood collection method using the blood collection unit according to the present invention.

### Mode for carrying out the invention

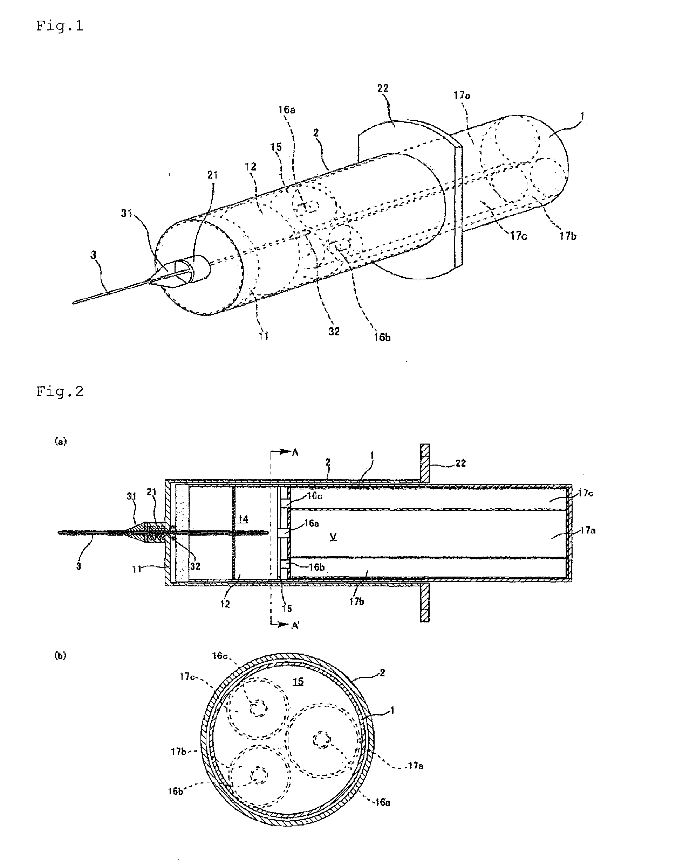

Referring to FIG. 1 and FIGS. 2A and 2B, 1 represents the vacuum blood collection tube which constitutes the blood collection unit according to the present invention. A vacuum blood collection tube 1 is the bottomed tubular body, and the open end facing the bottom is hermetically sealed by a plug body 11 in order to maintain the inner space of the tubular body at the prescribed reduced pressure V. Meanwhile, the plug body 11 is made of a material having high sealing performance, for example a rubber membrane.

In addition to the vacuum blood collection tube 1, the blood collection unit has a bottomed cylindrical holder 2 having the open end which allows insertion from the side of the plug body 11 of the vacuum blood collection tube 1, and a blood collection needle 3 made of the hollow metal narrow tube. A flange part 22 is provided at a peripheral part of the open end of the holder 2. In order to prevent wobble of the entire blood collection unit at the time of blood collection, the flange part 22 is s held by fingers of the person sampling the blood, for securing stability. FIG. 2A illustrates the side cross-sectional view of the blood collection unit, in which a female screw is cut and a needle-joining part 21 is provided on an approximately central part of the bottom of the holder 2. Meanwhile a needle hub 31 with the cut male screw is provided on the approximately central part in the longitudinal direction of the blood collection needle 3, and by the fact the fact that the needle hub 31 is screwed into the needle-joining part 21, one end of the blood collection needle 3 is exposed to the outside of the holder 2 from the needle-joining part 21, and the other end is fixed on the holder 2 in a state of being exposed in the axis direction inside the holder 2 from the needle-joining part 21. It should be noted that a fixation method for the blood collection needle 3 is not be limited to the screw type as mentioned above but may be a built-in type which allows attachment to and detachment from the holder 2 with one-touch operation. As shown in FIG. 2A, the other end of the blood collection needle 3 (hereinafter referred to as "the other end of the blood collection needle 3") exposed inside the holder 2 is covered with a sheath body 32 prior to the blood collection procedure.

It should be noted that a configuration in which the blood collection needle 3 is directly connected to the holder 2 is shown in an embodiment of the present invention, but a configuration in which the holder 2 is connected to a butterfly needle through a catheter may be shown (not shown).

In the tubular body constituting the vacuum blood collection tube 1, a plurality of inner spaces 17a, 17b and 17c into which the blood is collected are formed, and as shown in FIG. 2A, an accumulation area 14 in which a prescribed reduced pressure V is maintained is provided between the plug body 11 and the inner spaces 17a, 17b and 17c. Each of the inner spaces 17a, 17b and 17c has a prescribed capacity according to the purposes of the plural different blood test items and is divided by partitions so that the prescribed reduced pressure can be maintained. The inner spaces 17a, 17b and 17c may be formed so that the prescribed capacity is secured and the length in the shorter length direction is adjusted to be the same as the length in the longitudinal direction. Their lengths in the longitudinal direction are the same, thereby processing can be facilitated and production efficiency is improved.

The accumulation area 14 includes a stretch membrane 12, a detachment mechanism 13, a fixation membrane 15 and an inner wall surface of the tubular body. As shown in FIG. 2B, the accumulation area 14 is communicated with the inner spaces 17a, 17b and 17c through communication holes 16a, 16b and 16c which are provided on the fixation membrane 15. The stretch membrane 12 allows insertion of the other end of the blood collection needle 3 punctured into the plug body 11. Meanwhile the fixation membrane 15 is positioned facing the stretch membrane 12 in a direction toward the inner spaces 17a, 17b and 17c and does not allow penetration of the blood collection needle 3.

In each of the inner spaces 17a, 17b and 17c, the prescribed reduced pressure is maintained before the blood collection procedure, as mentioned above. When e blood collection needle 3 is punctured into the arm of the blood collection subject, the blood flows into the accumulation area 14 by the differential pressure between the blood pressure of the blood collection subject and the pressures of the inner spaces 17a, 17b and 17c. The inside blood is accumulated in the accumulation area 14 while expanding the stretch membrane 12. Subsequently, the accumulated blood is separated and flows into each of the inner spaces 17a, 17b and 17c through the communication holes 16a, 16b and 16c. When the differential pressure is resolved, and the pressure between the blood pressure and the inner spaces 17a, 17b and 17c reaches a state of equilibrium, inflow of the blood is discontinued. Consequently, each reduced pressure is adjusted so that the prescribed capacity of collection is allowed.

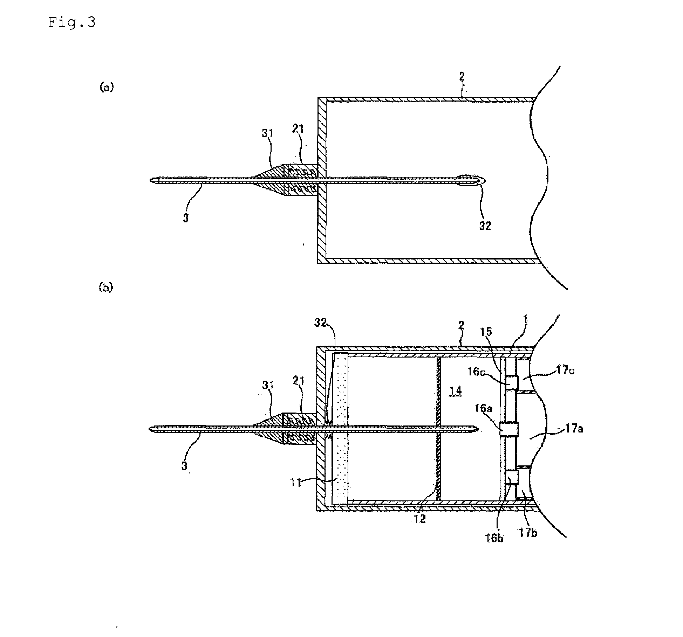

FIG. 3 (A) illustrates the side cross-sectional view of the state before the vacuum blood collection tube 1 is inserted into the holder 2 with the fixed blood collection needle 3. The other end of the blood collection needle 3 is covered with a sheath body 32. The sheath body 33 is made of an elastic material like a rubber. In this state, the vacuum blood collection tube 1 is inserted into the holder 2 from the open end of the holder 2, and the state following this is shown FIG. 3B. When the sheath body 32 is put into the plug body 11 by inserting the vacuum blood collection tube 1 into the holder 2, it is pierced by the other end of the blood collection needle 3 and pushed in an insertion direction of the vacuum blood collection tube 1 by the plug body 11 while the blood collection needle 3 is slid. When the plug body 11 reaches a vicinity of the bottom of the holder 2 (needle-joining part 21), it contracts like an accordion. Meanwhile the another end of the blood collection needle 3 piercing the sheath body 32 penetrates the plug body 11, then also penetrates the stretch membrane 12 and projects in the accumulation area 14.

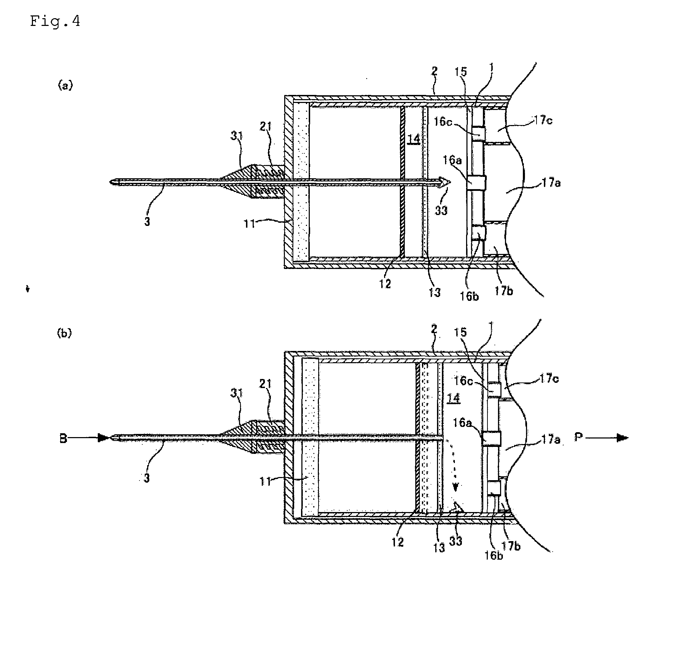

Although the configuration using the conventional blood collection needle (i.e. the blood collection needle 3 is covered with the sheath body 32) is shown in FIGS. 3A and 3B, it may also be a configuration illustrated in the following FIGS. 4A and 4B.

FIGS. 4A and 4B illustrate states before and after the blood collection procedure in a case that the other end of the blood collection needle 3 is occluded by an occlusion member 33. FIG. 4A illustrates a state before the blood collection procedure. Prior to the blood collection procedure, the other end of the blood collection needle 3 is punctured by the plug body 11 in a state of being occluded by the occlusion member 33, and is inserted into the stretch membrane 12 and the detachment mechanism 13 in the accumulation area 14.

The detachment mechanism 13 should be constituted so that when the occlusion member 33 enters in a direction of each of the inner spaces 17a, 17b and 17c from the plug body 11 side, it passes through the detachment mechanism 13, but once it has passed, the occlusion member 33 cannot pass through the detachment mechanism 13. For example, the tip of the occlusion member 33 is formed into an acuminated umbrella-like cone, and its bottom part should be formed into the after-mentioned hook shape which is caught on a mesh, so-called "barb structure". That is, the detachment mechanism 13 is formed into a mesh structure made from the elastic material like a rubber, and a mesh size should be smaller than the bottom of the cone of the occlusion member 32. When the occlusion member 33 is inserted from the tip of the cone, the mesh is broadened in association with pass of the occlusion member 33 and allows the pass, but once the occlusion member 33 has passed, in a case that the occlusion member 33 is returned to the reverse direction opposite to a direction of the pass, the occlusion member 33 is latched on the mesh by the hook-shaped "barb structure" formed on the bottom of the cone, and the occlusion member 33 can be detached by drag of the detachment mechanism 13.

FIG. 4B illustrates a state in which the occlusion member 33 is detached during the blood collection procedure. When the one end of the blood collection needle 3 is punctured into the arm of the blood collection subject, the blood inflows in a direction of Arrow B in the figure by the differential pressure between the blood pressure and the pressure in the accumulation area 14. At this time, the vacuum blood collection tube 1 is drawn in the direction of the open end of the holder 2 i.e. a direction of Arrow P in the figure, then the detachment mechanism 13 also moves in the direction of Arrow P. Since the blood collection needle 3 is fixed by the needle-joining part 21 of the holder 3, the occlusion member 33 attached to the other end of the blood collection needle 3 is detached by the detachment mechanism 13, and the blood flows in the accumulation area 14 from the another end of the blood collection needle 3. In order to prevent the detached occlusion member 33 from occluding the communication holes 16a, 16b and 16c by inflow of the blood, the occlusion member 33 may have a prescribed weight for resisting the blood flow, and may have a connecting body which connects to the blood collection needle 3 even after the occlusion member 33 is detached from the other end of the blood collection needle 3 (not shown).

In the blood collection unit shown in FIGS. 4A and 4B, as mentioned above, the vacuum blood collection tube 1 is merely drawn in the direction of the open end of the holder 2 after the one end of the blood collection needle 3 is punctured into the blood vessel of the blood collection subject, and thus there is no conventional event in which the blood collection needle moves through the influence of pushing force by inserting and pushing the vacuum blood collection tube from the open end of the holder, resulting in being able to reduce the burden on the blood collection subject. In addition, when the sheath body 32 shown in FIGS. 3A and 3B is used, contraction stress of the contracted sheath body 32 causes rebound resilience, resulting in kickback that is a phenomenon where the blood collection needle 3 is pushed out in a direction of the tip. However kickback is prevented by a constitution as shown in FIGS. 4A and 4B, and thus more advantageous blood collection unit can be provided.

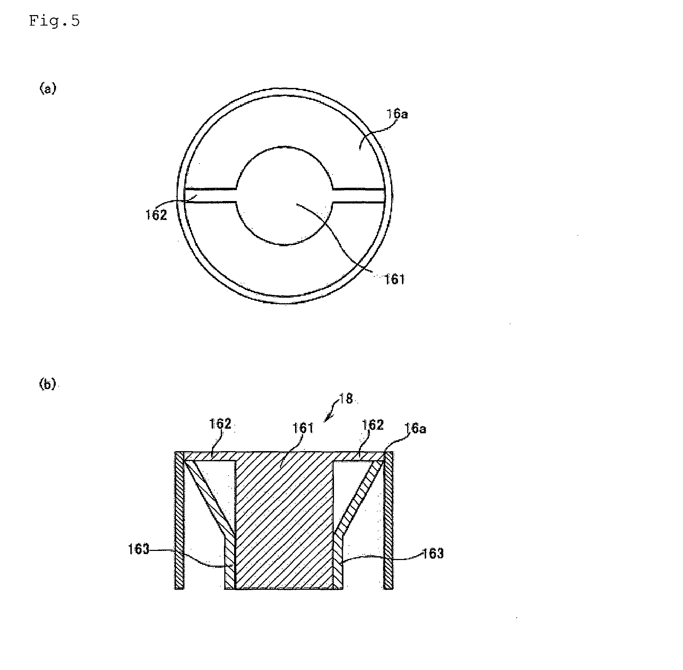

In order to prevent the blood which has been once collected in each of the inner spaces 17a, 17b and 17c from the communication holes 16a, 16b and 16c from flowing back to the accumulation area 14, the non-return mechanism which occludes the communication holes 16a, 16b and 16c should be provided. FIGS. 5A, 5B and 5C illustrate the non-return mechanism provided on the communication holes 16a, 16b and 16c. However FIGS. 5A, 5B and 5C illustrate one example of one non-return mechanism, and the blood collection subject matter is not limited to this constitution. Consequently, the non-return mechanism may also be any mechanism which is well-known non-return mechanism and compatible with the vacuum blood collection tube 1 according to the present invention, other than the constitution of FIGS. 5A, 5B and 5C.

FIG. 5A illustrates the top view of the non-return mechanism, and FIG. 5B illustrates its side cross-sectional view. It should be noted that FIGS. 5A, 5B and 5C will be explained by taking the communication hole 16a as an example, because the communication holes 16a, 16b and 16c are equipped with the identically-constituted non-return mechanisms.

The non-return mechanism includes a cylindrical stem body 161 positioned nearly in the middle of the communication hole 16a. An upper face of the stem body 161 is mounted so as to form a nearly flat face with the fixation membrane 15 (not shown in FIGS. 5A, 5B and 5C) with the communication hole 16a opening, and bridges 162 respectively extends in a radial direction on the upper face of the stem body 161 at an interval of about 180° and is fixed on a top of an inner wall of the communication hole 16a. As shown in FIG. 5B, the communication hole 16a is connected to a valve body 163 made of the elastic material. The valve body 163 opens a connecting part with the communication hole 16a so that its size is the same as the diameter of the communication hole 16a, and surrounds the stem body 161 in a tapered shape from the connecting part in a direction of inflow of the blood (in an axial direction of the stem body 161). This tapered valve body 163 contacts the stem body 161 nearly in the middle of the flank in the axial direction of the stem body 161, and at the lower part below the contacted area, the valve body is bent so as to be closely-attached to the stem body 161 along its flank. Consequently, between the valve body 163 and the communication hole 16a, a space where the valve body 163 can move in a radial direction of the communication hole 16a is formed. Due to such constitution of the valve body 163, the communication hole 16a is occluded when the valve body 163 is not pressured, so that both the inflow from the accumulation area 14 to the inner space 17a and the back-flow from the inner space 17a to the accumulation area 14 can be prevented. Meanwhile, as shown in FIG 5C, when the blood flows into the inner space 17a, a part of the valve body 163 closely-attached to the flank of the stem body 161 is pressured in a direction of diameter expansion by the differential pressure, and the valve body 163 moves to the space in which it can move, so that a space where the blood flows from the accumulation area 14 to the inner space 17a is formed as shown by Arrow B in the figure. When the differential pressure disappears and the state of equilibrium state is reached, the diameter of the diameter-expanded part is shortened again by elastic restoring force of the valve body 163, and the valve is restored and the back-flow of the blood is prevented.

When inflow of the blood is discontinued, a sealant is included in the accumulation area 14 from the blood collection needle 3 to seal the communication holes 16a, 16b and 16c, as mentioned below. Even if the vacuum blood collection tube 1 is drawn from the holder 2, the blood does not flow back into the accumulation area 14 by the sealant. It should be noted that the stretch membrane 12 and the fixation membrane 15 are made of a material that does not allow exudation of the inside blood and the included sealant.

After the sealant is included, the entire tubular body of the vacuum blood collection tube 1, or each of the inner spaces 17a, 17b and 17c constituting the tubular body may be respectively drawn for the inspection (not shown).

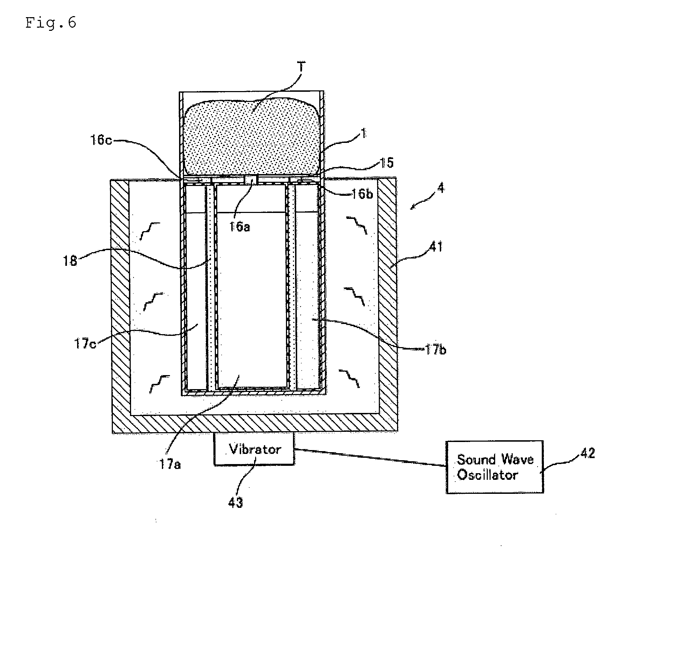

FIG. 6 illustrates a schematic view of a state in which the vacuum blood collection tube 1 according to the present invention is put into the stirrer. As explained in FIG. 1 and FIGS. 2A and 2B, the vacuum blood collection tube 1 according to the present invention has the plural inner spaces 17a, 17b and 17c therein, and each of them houses the collected blood for the different examination purposes. In a case that tests for example biochemistry, blood glucose level, blood count, etc., are simultaneously conducted, in blood sugar measurement and blood count, anticoagulation should be conducted, therefore previous preparations such as application of an anticoagulant agent in the inner space are carried out, and after the blood collection procedure, the sample should be immediately stirred by upside-down flipping or the stirrer, but in biochemistry, a serum is collected, therefore a coagulation accelerant and a separating agent are prepared without stirring. Consequently, in the constitution of the vacuum blood collection tube 1 according to the present invention, anticoagulation and coagulation which compete against one another should be simultaneously conducted.

In the present invention, the circumference of at least one of the plural inner spaces 17a, 17b and 17c was constituted to be coated by an acoustic wave absorber 18, and the collected blood in each of the inner spaces other than the inner space coated with the acoustic wave absorber 18 was stirred by stirring through acoustic wave oscillation. After the blood collection procedure is completed, a sealant T is suctioned from the blood collection needle 3, and the sealant T is included in the accumulation area 14. The sealant T may be, for example, any expandable resin which can be suctioned from the blood collection needle 3. The vacuum blood collection tube 1 in which the communication holes 16a, 16b and 16c are sealed by the sealant T is constituted so that it can be drawn together with the sealant T from the holder 2. The drawn vacuum blood collection tube 1 is put into a stirred vessel 41 of a stirrer 4 and transmits vibration to the stirred vessel through a vibrator 43 by a sound wave oscillated from a sound wave oscillator 42, and this vibration stirs the blood and the anticoagulant agent in the inner spaces other than the inner space coated with the acoustic wave absorber 18.

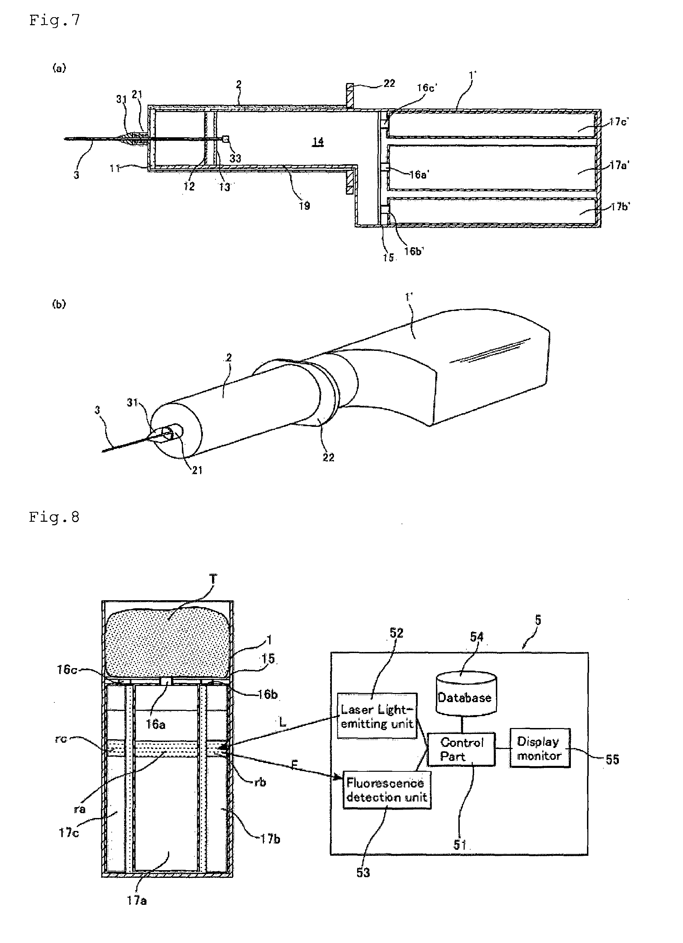

FIGS. 7A and 7B illustrate the embodiment where the vacuum blood collection tube 1 explained in FIG. 1 and FIGS. 2A and 2B are plate-like shaped. FIG. 7A illustrates a top schematic view of a state where a vacuum blood collection tube 1' of the embodiment is inserted into the holder 2. In the vacuum blood collection tube 1', a portion of the tubular body is formed into a plate-like shape by arranging the inner spaces 17a', 17b' and 17c' on the same plane in the shorter length direction of each inner space. Consequently, the accumulation area 14 is a space which is transversely long in the arranged direction. In the present embodiment, the plate-like shaped tubular body is exposed outside from the holder 2, and in the holder 2, only an inlet tube 19 which is formed in a direction of the tip of the tubular body can be inserted. In addition, a configuration in which the inlet tube 19 is not formed, the holder is deformed so that the entire tubular body i.e. the entire vacuum blood collection tube 1' can be inserted, and the entire vacuum blood collection tube 1' is inserted into the holder, may be taken (not shown).

As seen in the vacuum blood collection tube 1', the plate-like shaped portion of the tubular body allows efficient stack and transportation when the vacuum blood collection tube 1' is transferred to the inspection institute or the like. This is convenient particularly when a large amount of blood is collected in health check or the like at a clinic without examination equipments, or when the blood is collected at the temporary hospital in a disaster site, a battlefield, etc. and transferred to the inspection institute.

FIG. 7B is a further variation of FIG. 7A, wherein the plate-like shaped tubular body which is exposed from the holder 2 is flexible and made of a material which can be bent according to a curvature of the arm on which the tubular body is provided. Such a constitution allows stabilization of the vacuum blood collection tube 1' for collecting the blood, so that physical burdens of the blood collection subject and work burdens of the person who samples the blood can be further reduced. In addition, a constitution where a finger flange such as a trigger is provided on the holder 2, and this is pulled, thereby the vacuum blood collection tube 1' is moved, may be taken (not shown).

FIG. 8 illustrates the block configuration diagram of the device for discriminating test methods according to the present invention. As shown in FIG. 6, since a plurality of the blood samples for the different examination purposes are collected in the same tubular body in the vacuum blood collection tube 1 according to the present invention, it should be discriminated what inner space housing the blood and what kind of test are used for the inspection.

In the present invention, in order to discriminate the test items through the use of the device for discriminating test methods 5 using fluorescence reaction in the present invention, fluorescent agents ra, rb and rc exhibiting different fluorescence reactions corresponding to each of the inner spaces 17a, 17b and 17c are attached by a manner such as coating and pasting, when positions where each of the inner spaces 17a, 17b and 17c in the vacuum blood collection tube 1 can be discriminated from outside is irradiated with a prescribed light beam. In the embodiment, a device performing irradiation with a laser light will be explained, but the embodiment is not limited to this. In the device for discriminating test methods 5, a laser light-generating unit 52 is activated by a control part 51, and the laser light-generating unit 52 irradiates any of the fluorescent agents ra, rb and rc with a laser light L. The fluorescent agents irradiated with the laser light L respectively exhibit their own fluorescence reaction F. That is, they exhibit different fluorescence reactions depending on the fluorescent agents ra, rb and rc. A fluorescence detection unit 53 receives the fluorescence reaction F, a test method corresponding to each fluorescence reaction F is read out by a database 54 in the control part 51, and the read test method is displayed on a display monitor 55. The test method of the blood collected in each of the inner spaces 17a, 17b and 17c from the vacuum blood collection tube 1 can be discriminated by the device for discriminating test methods 5. In addition, the control part 51 may be any computer which includes a well-known CPU, memory, etc. and on which the computer program working each constituent element of the device for discriminating test methods 5 as mentioned above is provided. In addition, the device for discriminating test methods 5 may be connected to a testing apparatus and include function where the control part 51 actuates the testing apparatus (not shown) on the basis of the test method displayed on the display monitor 55.

Incidentally, in relation to the tubular bodies of the vacuum blood collection tube 1 illustrated in FIG. 1 to FIG. 8, the embodiment where the plural inner spaces 17a, 17b and 17c are integrally formed was shown, but the actual blood test items are various and their combinations vary according to the purpose of the blood test. When the tubular bodies s having the plural inner spaces 17a, 17b and 17c are integrally formed, the patterns of these combinations are simulated and the tubular bodies should be formed to the number of the patterns. In cases of combination patterns relatively frequently used, the integrally formed tubular bodies may be used, but in cases of integral tubular bodies partly combined with infrequently used patterns, the tubular bodies are not used unless the combination of the test items are required, and the inner spaces corresponding to other combined test items may spoil. There, in the case of such combinations, a part or whole of the inner space should be constituted to be detachable as cartridge-type tubular bodies if necessary.

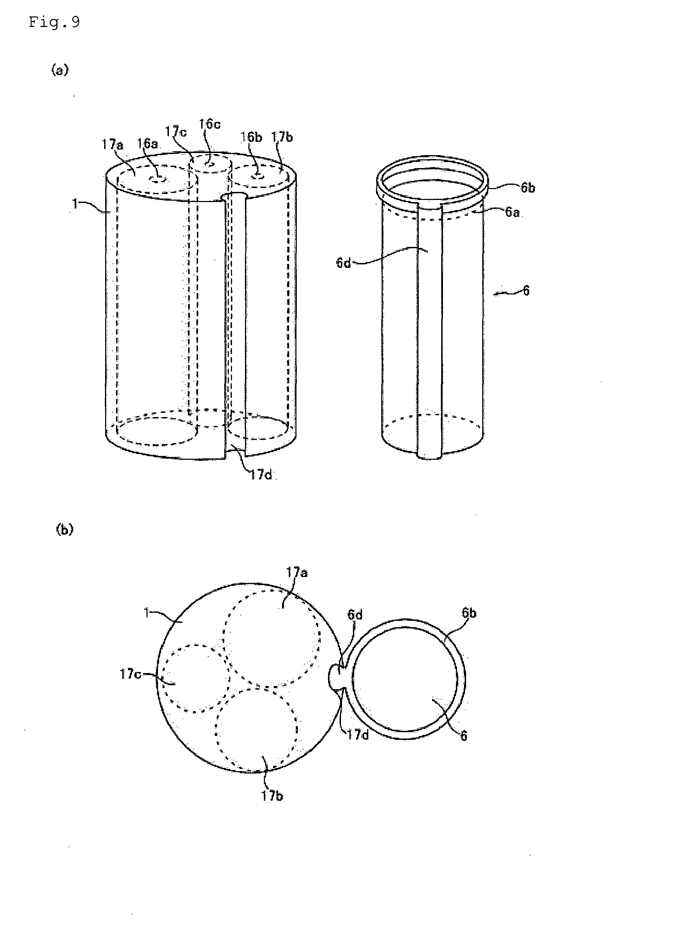

FIG. 9A illustrates the perspective views of a cartridge-type tubular body 6 which has bottom and an open end on its upper part, and a tubular body 1 capable of engaging with the cartridge-type tubular body 6, and FIG. 9B illustrates a bottom view in which the tubular body 1 is engaged with the cartridge-type tubular body 6. A guide groove 17d along an axial direction of the tubular body 1 is provided on a lateral surface of the tubular body 1. In the guide groove 17d has a cross-sectional surface which is shaped so that its groove width is broaden toward a central axis of the tubular body 1. Meanwhile, the cartridge-type tubular body 6 has a circular groove 6b with a U-shaped cross-sectional surface, in which the diameter of its top marginal part is more widely formed than the main body of the cartridge-type tubular body 6 so as to be attached to the fixation membrane 15 (not shown in FIGS. 9A and 9B), as mentioned below. Additionally, the cartridge-type tubular body 6 is occluded by an occlusion member 6a which occludes the open end on a somewhat lower position than the circular groove 6b before it is attached to the fixation membrane 15. The lateral surface of the cartridge-type tubular body 6 is provided with a guide rib 6d the cross-sectional surface of which has a shape so that it engages with the guide groove 17d of the tubular body 1.

The guide rib 6d is slidably fitted to the guide groove 17d of the tubular body 1 from the open end side of the cartridge-type tubular body 6 in a direction from the bottom of the tubular body 1 to the accumulation area 14 (not shown in FIGS. 9A and 9B), thereby the tubular body 1 can engage with the cartridge-type tubular body 6 in parallel. When the tubular body 1 engages with the cartridge-type tubular body 6 in parallel, the bottom face takes a shape shown in FIG. 9B. In the embodiment, a configuration in which the guide groove 17d engages with the guide rib 6d was exemplified, but the embodiment is not limited to this configuration unless it departs from Claims. That is, if the tubular body 1 can stably engage with the cartridge-type tubular body 6 without backlash, for example the guide groove and the guide rib may be positioned in a direction perpendicular to the axial direction, and the guide groove may be shaped to be wavy line from the top view (all are not shown).

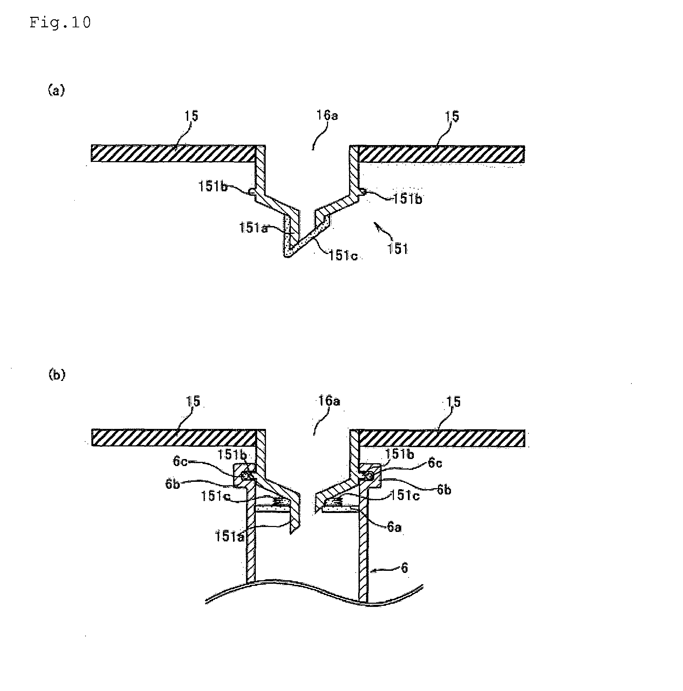

FIGS. 10A and 10B illustrate the side cross-sectional view of an attachment part 151 which attaches the cartridge-type tubular body 6 to the fixation membrane 15. Although the attachment part 151 may be provided on any communication hole, the explanation will be carried out on the attachment part provided on the communication hole 16a, as a matter of convenience for explanation, in the embodiment. FIG. 10A illustrates a drawing of the attachment part 151 before the cartridge-type tubular body 6 is attached. The attachment part 151 includes a connecting tube which connects the communication hole 16a to the cartridge-type tubular body 6. This connecting tube is shaped so that its lower part slants in a funnel shape from near the center of the axial direction in a direction of blood flow in the blood collection procedure. Furthermore a sharpening tubular puncture body 151a is formed on the tip. Before the cartridge-type tubular body 6 is attached, the puncture body 151a is covered with an elastic covering material 151c. When the cartridge-type tubular body 6 is not attached, this elastic covering material 151c can prevent the blood accumulated in the accumulation area 14 from leaking outside from the attachment part 151. A circular protrusion 151b is formed above a part where the connecting tube is shaped in a funnel shape.

FIG. 10B illustrates a drawing of a state in which the cartridge-type tubular body 6 is attached to the attachment part 151. As explained in FIGS. 9A, 9B and 9C, the circular groove 6b of the cartridge-type tubular body 6 is fit and attached to the circular protrusion 151b. It should be noted that an O-shaped ring 6c may be inserted to the circular groove 6b to prevent air or the like from entering from the outside. At the same time as the attachment, the occlusion member 6a of the cartridge-type tubular body 6 pushes out the elastic covering material 151c by contraction to expose the puncture body 151a, and this exposed puncture body 151a punctures the occlusion member 6a to communicate the communication hole 16a to the cartridge-type tubular body 6. As mentioned above, by the fact that one part of the tubular body is subsequently made to be detachable, the tubular body which can respond to various test items can be freely attached as appropriate to easily allow formation of the tubular body with no waste.

Although one part of the tubular body is made to be detachable by the cartridge-type tubular body in the above embodiment, every tubular body may also be constituted as a cartridge-type tubular body to make it detachable (not shown). In this case, any of the guide groove or the guide rib may be provided on the lateral surface of each cartridge-type tubular body so that they can engage with each other in parallel.

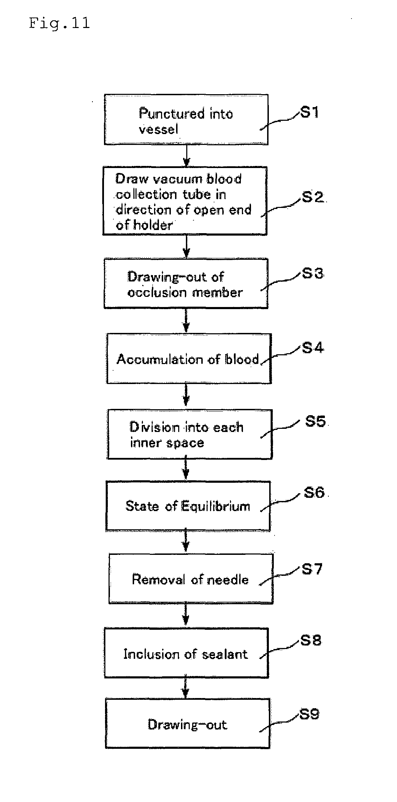

FIG. 11 illustrates the flow diagram which shows the procedure of the blood collection method using the blood collection unit according to the present invention. Hereinafter, explanation will be given with reference to FIG. 1, FIGS. 2A and 2B and FIGS. 3A and 3B. Prior to the blood collection procedure, the other end of the blood collection needle 3 is occluded by the occlusion member 32. In this state, the one end of the blood collection needle 3 is punctured into the blood vessel of the blood collection subject (S1). The vacuum blood collection tube 1 is drawn from the holder 2 in the direction of the open end of the holder 2 (S2), and the occlusion member 32 is drawn by the detachment mechanism 13 (S3). Because of the differential pressure between the pressure of the blood suctioned from the blood collection needle 3 and the pressure in the accumulation area 14 inside the vacuum blood collection tube 1 previously decompressed, the blood flows into the accumulation area 14, expands the stretch membrane 12 and is accumulated therein (S4). The accumulated blood is divided into each of the inner spaces 17a, 17b and 17c through the communication holes 16a, 16b and 16c (S5). When the differential pressure is released and the pressure between the blood pressure and the inner spaces 17a, 17b and 17c reaches a state of equilibrium (S6), inflow of the blood is discontinued, and thus the blood collection needle 3 is removed from the vessel of the blood collection subject (S7). Since, at this time, the blood in the accumulation area 14 outflows to the inner spaces 17a, 17b and 17c, the sealant T is sealed into the accumulation area 14 from the blood collection needle 3 (S8). When the sealant T is sufficiently sealed, the vacuum blood collection tube 1 is drawn from the holder 2 in the direction of the open end of the holder 2 and the blood collection procedure is terminated.

According to this method, once the blood collection needle 3 has been punctured into the vessel, the blood collection needle 3 does not move in the direction of puncture until the blood collection is discontinued, and thus distresses of the blood collection subject can be relieved.

### Reference Signs List

- 1: Vacuum blood collection tube
- 2: Holder
- 3: Blood collection needle
- 4: Stirrer
- 5: Device for discriminating test methods
- 6: Cartridge type tubular body
- 11: Plug body
- 12: Stretch membrane
- 13: Detachment mechanism
- 14: Accumulation area
- 15: Fixation membrane
- 16a, 16b, 16c: Communication hole
- 17a, 17b, 17c: Inner space
- 18: Acoustic wave absorber
- 19: Injection part
- 21: Needle-joining part
- 22: Flange part
- 31: Needle hub
- 32: Occlusion member

## Claims

1. A vacuum blood collection tube (1) which comprises a bottomed tubular body having inner spaces (17a, 17b, 17c) with a prescribed capacity and a plug body (11) which hermetically seals an open end of the tubular body in order to maintain the inner spaces (17a, 17b, 17c) at a prescribed reduced pressure, wherein
a blood is collected in the inner spaces (17a, 17b, 17c) of the tubular body by a differential pressure between a blood pressure and a pressure of the inner spaces (17a, 17b, 17c) via a hollow blood collection needle (3) one end of which is punctured into a blood vessel and the other end of which is punctured into the plug body (11),
the inner space is divided into a plurality of inner spaces (17a, 17b, 17c) by partitions, each of the inner spaces (17a, 17b, 17c) has the prescribed capacity and maintains a prescribed reduced pressure depending on purposes of various blood tests, and the blood of an amount depending on the purposes of the various blood tests is collected in each of the inner spaces (17a, 17b, 17c) via the blood collection needle (3) punctured into the blood vessel, **characterized in that**
the tubular body has, between the plug body (11) and the plural inner spaces (17a, 17b, 17c), a stretch membrane (12) which allows insertion of the other end of the blood collection needle (3) punctured into the plug body (11), a fixation membrane (15) which is positioned facing the stretch membrane (12) in a direction toward each of the inner spaces (17a, 17b, 17c) and does not allow penetration of the other end of the blood collection needle (3), and an accumulation area (14) for the blood which includes an inner wall surface of the tubular body and maintains the prescribed reduced pressure, the fixation membrane (15) is provided with communication holes (16a, 16b, 16c) which communicate the accumulation area (14) to each of the inner spaces (17a, 17b, 17c), and when the blood flows into the accumulation area (14) from the other end of the blood collection needle (3) inserted into the stretch membrane (12), the blood accumulates in the accumulation area (14) while expanding the stretch membrane (12) and divided into each of the inner spaces (17a, 17b, 17c) through the communication holes (16a, 16b, 16c).

2. The vacuum blood collection tube (1) according to Claim 1, wherein each of the inner spaces (17a, 17b, 17c) is formed so that the prescribed capacity is secured and a length in a shorter length direction is adjusted to be the same as a length in a longitudinal direction.

3. The vacuum blood collection tube (1) according to Claim 1 or 2, comprising a device configured to check return which opens each of the communication holes (16a, 16b, 16c) by the differential pressure when the blood is divided into each of the inner spaces (17a, 17b, 17c) from the accumulation area (14), and which occludes each of the inner spaces (17a, 17b, 17c) when the blood collected in each of the inner spaces (17a, 17b, 17c) reaches a prescribed volume and the pressure between the blood pressure and the pressure in the inner spaces (17a, 17b, 17c) reaches a state of equilibrium.

4. The vacuum blood collection tube (1) according to any one of Claims 1 to 3, which is constituted so that the accumulation area (14) after inflow of the blood into each of the inner spaces (17a, 17b, 17c) allows inclusion of a sealant which seals the communication holes (16a, 16b, 16c) from the blood collection needle (3).

5. The vacuum blood collection tube (1) according to any one of Claims 1 to 4, wherein each of the inner spaces (17a, 17b, 17c) is arranged in parallel on the same plane in a shorter length direction, and the tubular body is formed into a plate-like shape.

6. The vacuum blood collection tube (1) according to any one of Claims 1 to 5, wherein a circumference of at least one inner space of the plural inner spaces (17a, 17b, 17c) is coated with an acoustic wave absorber (18).

7. The vacuum blood collection tube (1) according to any one of Claims 1 to 6, wherein fluorescent agents exhibiting different fluorescence reactions corresponding to each inner space (17a, 17b, 17c) are coated, when positions where each of the inner spaces (17a, 17b, 17c) can be discriminated from an outside of the tubular body are irradiated with a prescribed light beam.

8. The vacuum blood collection tube (1) according to any one of Claims 1 to 7, wherein a guide groove is provided on any one lateral surface and a guide rib capable of engaging with the guide groove is provided on the other lateral surface, in order to engage the tubular body with a cartridge-type bottomed tubular (6) body the open end of which is occluded by an occlusion member (32) inparallel, and the fixation membrane (15) includes the communication hole (16a, 16b, 16c) allowing attachment of the cartridge-type tubular body (6).

9. The vacuum blood collection tube (1) according to Claim 8, wherein the communication hole (16a, 16b, 16c) allowing attachment of the cartridge-type tubular body (6) includes a tubular device configured to perform puncture which is covered with the elastic covering material, and when the cartridge-type tubular body (6) is attached, the device configured to perform puncture is pushed out from the elastic covering material by contracting the elastic covering material due to the occlusion member (32), to thereby puncture the occlusion member (32) for communication between the communication holes (16a, 16b, 16c) and the cartridge-type tubular body (6).

10. The vacuum blood collection tube (1) according to Claim 8 or 9, wherein all the communication holes (16a, 16b, 16c) of the fixation membrane (15) allow attachment of the cartridge-type tubular body (6), and the cartridge-type tubular body (6) to be attached to each communication hole (16a, 16b, 16c) has any of the guide groove or the guide rib on its lateral surface, for parallel engagement.

11. A blood collection unit which includes the vacuum blood collection tube (1) of any one of Claims 1 to 10, the blood collection needle (3) the one end of which is punctured into the blood vessel and the other end of which is punctured into the plug body (11) which hermetically seals the open end of the tubular body constituting the vacuum blood collection tube (1), and a bottomed cylindrical holder (2) having an open end which allows insertion of the vacuum blood collection tube (1) and having a needle-joining part (21) which connects to the blood collection needle (3) on the other end bottom.

12. The blood collection unit according to Claim 11, wherein the blood collection needle (3) includes a sheath body which covers the other end and is pierced by the other end and contracted when the blood collection needle (3) is stuck into the plug body (11) of the vacuum blood collection tube (1) inserted into the holder (2) from the open end of the holder (2) connected to the needle-joining part (21), and the other end piercing the sheath body penetrates the plug body (11) and projects to the inside of the tubular body.

13. The blood collection unit according to Claim 11, wherein the blood collection needle (3) has an occlusion member (32) which occludes the other end of the blood collection needle (3) in the tubular body, and a device configured to perform detachment, which latches and detaches the occlusion member (32) from the blood collection needle (3) by drawing the vacuum blood collection tube (1) in a direction of the open end of the holder (2) is provided in the tubular body.

14. The blood collection unit according to any one of Claims 11 to 13, wherein the tube plate-like shaped vacuum blood collection tube (1) of Claim 5 includes an inlet tube with a size allowing insertion into the holder (2), and a plate-like shaped part is exposed outside from the holder (2).

15. A device for discriminating test methods (5) for the blood which includes a device configured to perform irradiation, which irradiates, with the prescribed light beam, the tubular body coated with given fluorescent agents of Claim 7,
a device configured to perform detection, which detects the respective fluorescence reactions of the fluorescent agents irradiated with the light beam,
a device configured to perform display, which reads out the test method of the blood collected in the respective inner spaces (17a, 17b, 17c) corresponding to the detected fluorescence reactions displays the test method read out.

## Patentansprüche

1. Vakuumunterstütztes Blutentnahmeröhrchen (1), das einen mit einem Boden versehenen, rohrförmigen Körper umfasst, der Innenräume (17a, 17b, 17c) mit einem vorgeschriebenen Aufnahmevermögen und einen Stopfenkörper (11) aufweist, der ein offenes Ende des rohrförmigen Körpers hermetisch dicht verschließt, um die Innenräume (17a, 17b, 17c) auf einem vorgeschriebenen verminderten Druck zu halten, wobei
sich über einen Druckunterschied zwischen einem Blutdruck und einem Druck der Innenräume (17a, 17b, 17c) Blut in den Innenräumen (17a, 17b, 17c) des rohrförmigen Körpers über eine hohle Blutentnahmenadel (3) ansammelt, von der ein Ende in ein Blutgefäß eingestochen ist und deren anderes Ende in den Stopfenkörper (11) eingestochen ist,
der Innenraum durch Trennwände in mehrere Innenräume (17a, 17b, 17c) unterteilt ist, wobei jeder der Innenräume (17a, 17b, 17c) das vorgeschriebene Aufnahmevermögen hat und je nach Einsatzzweck verschiedener Bluttests einen vorgeschriebenen verminderten Druck aufrechterhält, und das Blut sich über die in das Blutgefäß eingestochene Blutentnahmenadel (3) je nach Einsatzzweck der verschiedenen Bluttests in einer Menge in jedem der Innenräume (17a, 17b, 17c) ansammelt, **dadurch gekennzeichnet, dass**
der rohrförmige Körper zwischen dem Stopfenkörper (11) und den mehreren Innenräumen (17a, 17b, 17c) eine Dehnmembran (12), die das Einführen des in den Stopfenkörper (11) eingestochenen anderen Endes der Blutentnahmenadel (3) ermöglicht, eine Fixierungsmembran (15), die der Dehnmembran (12) in einer Richtung zu jedem der Innenräume (17a, 17b, 17c) zugewandt angeordnet ist und das Eindringen des anderen Endes der Blutentnahmenadel (3) nicht ermöglicht, und einen Ansammlungsbereich (14) für das Blut aufweist, der eine Innenwandoberfläche des rohrförmigen Körpers umfasst und den vorgeschriebenen verminderten Druck aufrechterhält, wobei die Fixierungsmembran (15) mit Verbindungsöffnungen (16a, 16b, 16c) versehen ist, die den Ansammlungsbereich (14) mit jedem der Innenräume (17a, 17b, 17c) verbinden, und, wenn das Blut ausgehend vom anderen Ende der in die Dehnmembran (12) eingeführten Blutentnahmenadel (3) in den Ansammlungsbereich (14) einströmt, das Blut sich im Ansammlungsbereich (14) ansammelt, während dabei die Dehnmembran (12) gedehnt wird, und durch die Verbindungsöffnungen (16a, 16b, 16c) auf die jeweiligen Innenräume (17a, 17b, 17c) aufgeteilt wird.

2. Vakuumunterstütztes Blutentnahmeröhrchen (1) nach Anspruch 1, wobei die Innenräume (17a, 17b, 17c) jeweils so ausgebildet sind, dass das vorgeschriebene Aufnahmevermögen sichergestellt ist, und eine Länge in einer kürzeren Längenrichtung so eingestellt ist, dass sie einer Länge in einer Longitudinalrichtung entspricht.

3. Vakuumunterstütztes Blutentnahmeröhrchen (1) nach Anspruch 1 oder 2, mit einer Vorrichtung, die dazu ausgelegt ist, den Rückfluss zu prüfen, und die die Verbindungsöffnungen (16a, 16b, 16c) durch den Druckunterschied jeweils öffnet, wenn das Blut ausgehend vom Ansammlungsbereich (14) auf die jeweiligen Innenräume (17a, 17b, 17c) aufgeteilt wird, und die Innenräume (17a, 17b, 17c) jeweils verschließt, wenn das in einem jeweiligen Innenraum (17a, 17b, 17c) angesammelte Blut ein vorgeschriebenes Volumen erreicht und der Druck zwischen dem Blutdruck und dem Druck in den Innenräumen (17a, 17b, 17c) einen Gleichgewichtszustand erreicht.

4. Vakuumunterstütztes Blutentnahmeröhrchen (1) nach einem der Ansprüche 1 bis 3, das so gebildet ist, dass der Ansammlungsbereich (14) nach dem Einströmen des Blutes in die jeweiligen Innenräume (17a, 17b, 17c) die Einbeziehung eines Dichtmittels ermöglicht, das die Verbindungsöffnungen (16a, 16b, 16c) gegenüber der Blutentnahmenadel (3) abdichtet.

5. Vakuumunterstütztes Blutentnahmeröhrchen (1) nach einem der Ansprüche 1 bis 4, wobei die Innenräume (17a, 17b, 17c) jeweils parallel in derselben Ebene in einer kürzeren Längenrichtung angeordnet sind und der rohrförmige Körper zu einer plattenartigen Gestalt ausgeformt ist.

6. Vakuumunterstütztes Blutentnahmeröhrchen (1) nach einem der Ansprüche 1 bis 5, wobei ein Umfang mindestens eines Innenraums der mehreren Innenräume (17a, 17b, 17c) mit einem Schallwellenabsorber (18) beschichtet ist.

7. Vakuumunterstütztes Blutentnahmeröhrchen (1) nach einem der Ansprüche 1 bis 6, wobei fluoreszierende Stoffe, die unterschiedliche Fluoreszenzreaktionen zeigen, entsprechend jedem Innenraum (17a, 17b, 17c) aufgebracht sind, wenn Positionen, an denen die Innenräume (17a, 17b, 17c) jeweils von außerhalb des rohrförmigen Körpers unterschieden werden können, mit einem vorgeschriebenen Lichtstrahl bestrahlt werden.

8. Vakuumunterstütztes Blutentnahmeröhrchen (1) nach einem der Ansprüche 1 bis 7, wobei eine Führungsnut an einer beliebigen Seitenfläche vorgesehen ist, und eine Führungsrippe, die mit der Führungsnut in Eingriff gelangen kann, an der anderen Seitenfläche vorgesehen ist, um den rohrförmigen Körper mit einem kartuschenartigen, mit Boden versehenen rohrförmigen Körper (6) in Eingriff gelangen zu lassen, dessen offenes Ende parallel durch ein Verschlusselement (32) verschlossen ist, und die Fixierungsmembran (15) die Verbindungsöffnung (16a, 16b, 16c) aufweist, die eine Anbringung des kartuschenartigen rohrförmigen Körpers (6) ermöglicht.

9. Vakuumunterstütztes Blutentnahmeröhrchen (1) nach Anspruch 8, wobei die Verbindungsöffnung (16a, 16b, 16c), die die Anbringung des kartuschenartigen rohrförmigen Körpers (6) ermöglicht, eine rohrförmige Vorrichtung aufweist, die dazu eingerichtet ist, einen Einstechvorgang auszuführen, und die mit einem elastischen Abdeckmaterial bedeckt ist, und, wenn der kartuschenartige rohrförmige Körper (6) angebracht ist, die zum Ausführen des Einstechvorgangs ausgelegte Vorrichtung aus dem elastischen Abdeckmaterial ausgestoßen wird, indem sich das elastische Abdeckmaterial aufgrund des Verschlusselements (32) zusammenzieht, um dadurch das Verschlusselement (32) zur Herstellung einer Verbindung zwischen den Verbindungsöffnungen (16a, 16b, 16c) und dem kartuschenartigen rohrförmigen Körper (6) zu durchstoßen.

10. Vakuumunterstütztes Blutentnahmeröhrchen (1) nach Anspruch 8 oder 9, wobei die Verbindungsöffnungen (16a, 16b, 16c) der Fixierungsmembran (15) jeweils die Anbringung des kartuschenartigen rohrförmigen Körpers (6) ermöglichen, und der an jeder Verbindungsöffnung (16a, 16b, 16c) anzubringende, kartuschenartige rohrförmige Körper (6) für einen parallelen Eingriff eine oder mehrere Führungsnuten oder Führungsrippen an seiner Seitenfläche aufweist.

11. Blutentnahmeeinheit, die das vakuumunterstützte Blutentnahmeröhrchen (1) nach einem der Ansprüche 1 bis 10, die Blutentnahmenadel (3), von der ein Ende in ein Blutgefäß und deren anderes Ende in den Stopfenkörper (11) eingestochen ist, der das offene Ende des das vakuumunterstützte Blutentnahmeröhrchen (1) bildenden rohrförmigen Körpers hermetisch dicht verschließt, und eine mit einem Boden versehene, zylindrische Halterung (2) mit einem offenen Ende aufweist, die das Einführen des vakuumunterstützten Blutentnahmeröhrchens (1) ermöglicht und ein Nadelverbindungsteil (21) aufweist, das eine Verbindung zur Blutentnahmenadel (3) am anderen Endboden herstellt.

12. Blutentnahmeeinheit nach Anspruch 11, wobei die Blutentnahmenadel (3) einen Ummantelungskörper aufweist, der das andere Ende bedeckt und vom anderen Ende durchstoßen wird und zusammengeschoben wird, wenn die Blutentnahmenadel (3) ausgehend vom offenen Ende der Halterung (2), die mit dem Nadelverbindungsteil (21) verbunden ist, in den Stopfenkörper (11) des in die Halterung (2) eingeführten, vakuumunterstützten Blutentnahmeröhrchens (1) eingesteckt ist, und das andere, den Ummantelungskörper durchstechende Ende den Stopfenkörper (11) durchdringt und in den Innenraum des rohrförmigen Körpers ragt.

13. Blutentnahmeeinheit nach Anspruch 11, wobei die Blutentnahmenadel (3) ein Verschlussteil (32) aufweist, welches das andere Ende der Blutentnahmenadel (3) im rohrförmigen Körper verschließt, und eine Vorrichtung, die dazu ausgelegt ist, einen Ablösevorgang auszuführen und das Verschlussteil (32) an der Blutentnahmenadel (3) arretiert bzw. es von dieser löst, indem das vakuumunterstützte Blutentnahmeröhrchen (1) in eine Richtung des offenen Endes des Halters (2) gezogen wird, im rohrförmigen Körper vorgesehen ist.

14. Blutentnahmeeinheit nach einem der Ansprüche 11 bis 13, wobei das rohrförmige, plattenartige vakuumunterstützte Blutentnahmeröhrchen (1) von Anspruch 5 ein Einlassrohr mit einer Größe aufweist, die das Einführen in die Halterung (2) ermöglicht, und ein plattenartig geformter Teil außerhalb der Halterung (2) freiliegt.

15. Vorrichtung für unterscheidungsfähige Testverfahren (5) für Blut, mit einer Vorrichtung, die dazu ausgelegt ist, eine Bestrahlung vorzunehmen, und die den rohrförmigen Körper, der mit bestimmten fluoreszierenden Stoffen nach Anspruch 7 beschichtet ist, mit einem vorgeschriebenen Lichtstrahl bestrahlt,
mit einer Vorrichtung, die dazu ausgelegt ist, eine Erfassung vorzunehmen, und die die jeweiligen Fluoreszenzreaktionen der mit dem Lichtstrahl bestrahlten fluoreszierenden Stoffe erfasst,
und mit einer Vorrichtung, die dazu ausgelegt ist, eine Anzeige vorzunehmen, und die das Testverfahren des in den jeweiligen Innenräumen (17a, 17b, 17c) angesammelten Blutes entsprechend den erfassten Fluoreszenzreaktionen ausliest und das ausgelesene Testverfahren anzeigt.

## Revendications

1. Tube de prélèvement sanguin sous vide (1) qui comprend un corps tubulaire avec fond comportant des espaces intérieurs (17a, 17b, 17c) d'une capacité prescrite et un corps de bouchon (11) qui rend étanche hermétiquement une extrémité ouverte du corps tubulaire afin de maintenir les espaces intérieurs (17a, 17b, 17c) à une pression réduite prescrite, dans lequel
un sang est prélevé dans les espaces intérieurs (17a, 17b, 17c) du corps tubulaire par une pression différentielle entre une pression sanguine et une pression des espaces intérieurs (17a, 17b, 17c) via une aiguille creuse de prélèvement sanguin (3) dont une extrémité est introduite dans un vaisseau sanguin et dont l'autre extrémité est introduite dans le corps de bouchon (11),
l'espace intérieur est divisé en une pluralité d'espaces intérieurs (17a, 17b, 17c) par des cloisonnements, chacun des espaces intérieurs (17a, 17b, 17c) a la capacité prescrite et maintient une pression réduite prescrite en fonction de buts de divers tests sanguins, et le sang d'une quantité dépendante des buts des divers tests sanguins est prélevé dans chacun des espaces intérieurs (17a, 17b, 17c) via l'aiguille de prélèvement sanguin (3) introduite dans le vaisseau sanguin, **caractérisé en ce que**
le corps tubulaire comporte, entre le corps de bouchon (11) et les multiples espaces intérieurs (17a, 17b, 17c), une membrane élastique (12) qui permet l'insertion de l'autre extrémité de l'aiguille de prélèvement sanguin (3) introduite dans le corps de bouchon (11), une membrane de fixation (15) qui est positionnée en face de la membrane élastique (12) dans une direction orientée vers chacun des espaces intérieurs (17a, 17b, 17c) et ne permet pas la pénétration de l'autre extrémité de l'aiguille de prélèvement sanguin (3), et une zone d'accumulation (14) pour le sang qui inclut une surface de paroi intérieure du corps tubulaire et maintient la pression réduite prescrite, la membrane de fixation (15) est pourvue de trous de communication (16a, 16b, 16c) qui font communiquer la zone d'accumulation (14) avec chacun des espaces intérieurs (17a, 17b, 17c), et lorsque le sang s'écoule dans la zone d'accumulation (14) à partir de l'autre extrémité de l'aiguille de prélèvement sanguin (3) insérée dans la membrane élastique (12), le sang s'accumule dans la zone d'accumulation (14) tout en étendant la membrane élastique (12) et est réparti dans chacun des espaces intérieurs (17a, 17b, 17c) via les trous de communication (16a, 16b, 16c).

2. Le tube de prélèvement sanguin sous vide (1) selon la revendication 1, dans lequel chacun des espaces intérieurs (17a, 17b, 17c) est formé de telle manière que la capacité prescrite soit assurée et qu'une longueur dans une direction de longueur plus courte est réglée pour être égale à une longueur dans une direction longitudinale.

3. Le tube de prélèvement sanguin sous vide (1) selon la revendication 1 ou 2, comprenant un dispositif configuré pour contrôler un retour qui ouvre chacun des trous de communication (16a, 16b, 16c) par la pression différentielle lorsque le sang est réparti dans chacun des espaces intérieurs (17a, 17b, 17c) depuis la zone d'accumulation (14), et qui obture chacun des espaces intérieurs (17a, 17b, 17c) lorsque le sang prélevé dans chacun des espaces intérieurs (17a, 17b, 17c) atteint un volume prescrit et que la pression entre la pression sanguine et la pression dans les espaces intérieurs (17a, 17b, 17c) atteint un état d'équilibre.

4. Le tube de prélèvement sanguin sous vide (1) selon l'une quelconque des revendications 1 à 3, qui est constitué de telle manière que la zone d'accumulation (14), après l'afflux du sang dans chacun des espaces intérieurs (17a, 17b, 17c), permet l'inclusion d'un produit d'étanchéité qui rend étanches les trous de communication (16a, 16b, 16c) par rapport à l'aiguille de prélèvement sanguin (3).

5. Le tube de prélèvement sanguin sous vide (1) selon l'une quelconque des revendications 1 à 4, dans lequel chacun des espaces intérieurs (17a, 17b, 17c) est disposé en parallèle sur le même plan dans une direction de longueur plus courte, et le corps tubulaire est constitué en forme de plaque.

6. Le tube de prélèvement sanguin sous vide (1) selon l'une quelconque des revendications 1 à 5, dans lequel une circonférence d'au moins un espace intérieur des multiples espaces intérieurs (17a, 17b, 17c) est revêtue d'un absorbeur d'ondes acoustiques (18).

7. Le tube de prélèvement sanguin sous vide (1) selon l'une quelconque des revendications 1 à 6, dans lequel des agents fluorescents présentant différentes réactions de fluorescence correspondant à chaque espace intérieur (17a, 17b, 17c) sont revêtus, lorsque des positions où chacun des espaces intérieurs (17a, 17b, 17c) peut être distingué par rapport à un extérieur du corps tubulaire sont irradiées par un faisceau lumineux prescrit.

8. Le tube de prélèvement sanguin sous vide (1) selon l'une quelconque des revendications 1 à 7, dans lequel une rainure de guidage est disposée sur une quelconque surface latérale et une nervure de guidage apte à s'emboîter dans la rainure de guidage est disposée sur l'autre surface latérale, afin d'emboîter le corps tubulaire dans un corps tubulaire avec fond de type cartouche (6) dont l'extrémité ouverte est obturée par un élément d'obturation (32) en parallèle, et la membrane de fixation (15) comprend les trous de communication (16a, 16b, 16c) permettant d'attacher le corps tubulaire de type cartouche (6).

9. Le tube de prélèvement sanguin sous vide (1) selon la revendication 8, dans lequel le trou de communication (16a, 16b, 16c) permettant d'attacher le corps tubulaire de type cartouche (6) comprend un dispositif tubulaire configuré pour effectuer un percement et recouvert du matériau de recouvrement élastique, et lorsque le corps tubulaire de type cartouche (6) est attaché, le dispositif configuré pour effectuer un percement est poussé hors du matériau de recouvrement élastique par contraction du matériau de recouvrement élastique en raison de l'élément d'obturation (32), pour percer ainsi l'élément d'obturation (32) afin de réaliser une communication entre les trous de communication (16a, 16b, 16c) et le corps tubulaire de type cartouche (6).

10. Le tube de prélèvement sanguin sous vide (1) selon la revendication 8 ou 9, dans lequel tous les trous de communication (16a, 16b, 16c) de la membrane de fixation (15) permettent d'attacher le corps tubulaire de type cartouche (6), et le corps tubulaire de type cartouche (6) devant être attaché à chaque trou de communication (16a, 16b, 16c) comporte soit la rainure de guidage soit la nervure de guidage sur sa surface latérale, en vue d'un emboîtement parallèle.

11. Unité de prélèvement sanguin qui comprend le tube de prélèvement sanguin sous vide (1) de l'une quelconque des revendications 1 à 10, l'aiguille de prélèvement sanguin (3) dont l'une extrémité est introduite dans le vaisseau sanguin et dont l'autre extrémité est introduite dans le corps de bouchon (11) qui rend étanche hermétiquement l'extrémité ouverte du corps tubulaire constituant le tube de prélèvement sanguin sous vide (1), et un support cylindrique avec fond (2) comportant une extrémité ouverte qui permet l'insertion du tube de prélèvement sanguin sous vide (1) et comportant une pièce de raccord d'aiguille (21) qui connecte l'aiguille de prélèvement sanguin (3) sur l'autre fond d'extrémité.

12. L'unité de prélèvement sanguin selon la revendication 11, dans laquelle l'aiguille de prélèvement sanguin (3) comprend un corps de gaine qui recouvre l'autre extrémité et est transpercé par l'autre extrémité et contracté lorsque l'aiguille de prélèvement sanguin (3) est enfichée dans le corps de bouchon (11) du tube de prélèvement sanguin sous vide (1) inséré dans le support (2) depuis l'extrémité ouverte du support (2) connectée à la pièce de raccord d'aiguille (21), et l'autre extrémité transperçant le corps de gaine pénètre dans le corps de bouchon (11) et s'avance dans l'intérieur du corps tubulaire.

13. L'unité de prélèvement sanguin selon la revendication 11, dans laquelle l'aiguille de prélèvement sanguin (3) comporte un élément d'obturation (32) qui obture l'autre extrémité de l'aiguille de prélèvement sanguin (3) dans le corps tubulaire, et un dispositif configuré pour effectuer un détachement, qui attache et détache l'élément d'obturation (32) de l'aiguille de prélèvement sanguin (3) en retirant le tube de prélèvement sanguin sous vide (1) dans une direction de l'extrémité ouverte du support (2), est disposé dans le corps tubulaire.

14. L'unité de prélèvement sanguin selon l'une quelconque des revendications 11 à 13, dans laquelle le tube de prélèvement sanguin sous vide (1) en forme de plaque de la revendication 5 comprend un tube d'entrée d'une taille permettant l'insertion dans le support (2), et une partie en forme de plaque est exposée à l'extérieur du support (2).

15. Dispositif destiné à distinguer des méthodes de test (5) pour le sang, qui comprend un dispositif configuré pour effectuer une irradiation, laquelle irradie, avec le faisceau lumineux prescrit, le corps tubulaire revêtu desdits agents fluorescents de la revendication 7,
un dispositif configuré pour effectuer une détection, lequel détecte les réactions de fluorescence respectives des agents fluorescents irradiés par le faisceau lumineux,
un dispositif configuré pour effectuer l'affichage, lequel extrait la méthode de test du sang prélevé dans les espaces intérieurs (17a, 17b, 17c) respectifs correspondant aux réactions de fluorescence détectées et affiche la méthode de test extraite.
